# EUROPEAN PATENT APPLICATION

(11) **EP 3 062 120 A1**
(43) Date of publication of application: **31.08.2016**
(21) Application number: 16152011.9
(22) Date of filing: 20.01.2016
(51) Int. Cl.: G01R 33/09, A61N 1/37, A61N 1/372, A61B 5/042, A61B 5/055

(54) **MAGNETIC FIELD SENSOR ARRANGEMENT**

(30) Priority: 26.02.2015 US 201562120909 P
(71) Applicant: BIOTRONIK SE & Co. KG, 12359 Berlin (DE)
(72) Inventor: HESS, James W., Aloha, OR Oregon 97003 (US); BERTHELSDORF, Richard, Dundee, OR 97115 (US)
(74) Representative: Galander, Marcus

(57) **Abstract**

The invention relates to a magnetic field sensor arrangement that comprises at least one sensing circuit comprising Giant magnetoresistive (GMR) elements. At least one of the GMR elements is at least partially housed in a shielding or flux concentrating first material that reduces or amplifies, respectively, the magnetic field impinging on them, while at least one other GMR element is not housed in such material, or is housed in a material having an opposite effect of the first material. The magnetic field sensor further comprises a measurement circuit that is configured for voltage measurement between different points of the GMR sensing circuit in order to derive the electric impedance between the different measurement points. The magnetic field sensor further comprises an evaluation circuit that is configured to evaluate the measured impedances and the relations of the measured impedances in order to derive a signal that indicates the level of an applied magnetic field.

## Description

The invention relates to a magnetic field sensor arrangement for sensing magnetic fields of different strengths. In particular, the invention relates to an implantable medical device, e.g. an implantable pulse generator such as a heart stimulator or a neurostimulator comprising a magnetic field sensor arrangement for sensing magnetic fields of different strengths. A further aspect of the invention relates to a method for determining the presence of a magnetic field.

For more than 45 years, manufacturers have used a reed switch in cardiac implants to set the implant into a mode of operation that is commonly referred to as the magnet mode. The reed switch is normally open, and is closed when a permanent magnet is brought into close proximity to the implant.

Magnetic Resonance Imaging (MRI) is a diagnostic tool that has become increasingly popular, and is typically contraindicated for pacemaker and implantable cardioverter/defibrillator (ICD) patients due to possible mechanical forces, heating of leads, permanent damage to the electronic circuit or inappropriate therapy (e.g. loss of sensing or pacing at the upper tracking rate (UTR)). MRI machines typically use a static magnetic field with field strength of 1.5T, although fields of 3T or higher may be present in newer machines. When it is exposed to such a field, a reed switch will remain closed, placing the device into its magnet mode. However, a reed switch is unable to differentiate between different ranges of the magnetic field strength.

The use of a Hall sensor to detect magnetic fields is a known alternative to reed switches. The Hall effect sensor has the advantage that its output voltage is proportional to the strength of the magnetic field, enabling determination of whether the magnetic field is from a normal permanent magnet or an MRI. US 6,937,906 discloses an implantable pacemaker being able to detect the MRI magnetic field, as well as automatically changing the mode of the device to "a second sensing mode less effected by the MRI interference signal".

A known alternative to a reed switch is a GMR sensor, see US 6,101,417, US 2008/01543442 A1 or US 2011/0202104.

A GMRs response to a magnetic field varies as the angle of the field changes relative to the GMR. To compensate for this multiple GMRs can be used mounted at various angles to each other. Using multiple GMRs results in more components, higher cost, and higher current consumption.

It is an object of the invention to provide magnetic filed sensor arrangement that allows reliable detection of magnetic fields of different strengths and orientations while keeping costs and energy consumption low.

According to the invention, this object is achieved by a magnetic field sensor arrangement that comprises at least one GMR sensing circuit comprising GMR elements. At least one of the GMR elements is at least partially housed in a shielding or flux concentrating first material that reduces or amplifies, respectively, the magnetic field impinging on them, while at least one other GMR element is not housed in such material or housed in a material having an opposite effect than the first material. The magnetic field sensor further comprises a measurement circuit that is configured for the voltage measurement at different points of the GMR sensing circuit for deriving the electric impedance between the different measurement points to derive a signal that indicates a level of an outer magnetic field.

In a preferred embodiment of the invention, the magnetic field sensor arrangement further comprises an evaluation circuit that is configured to evaluate the measured impedances and the relations of the measured impedances to derive a signal that indicates a level of an outer magnetic field.

Replacing the reed switch with a GMR and using that same GMR for MRI detection allows adding a feature to the implant while lowering the component cost of the device. In addition, the deletion of a mechanical element - the reed switch - is expected to enhance the reliability of the implant.

By combining the use of shielded and non-shielded GMR elements along with using multiple GMRs oriented at various angles relative to each other, the sensitivity of the GMR to the orientation of the GMR in relation to the magnetic field can be reduced, thus providing better detection / reducing the costs of the device.

By monitoring the overall resistance change of a GMR sensor circuit in a bridge configuration, higher level fields (MRI Detection) may also be detected using the same device.

This can result in fewer GMR components being used for a device, thus lowering its cost.

The GMR-based magnetic field sensor of the invention allows MRI detection in implants such as pacemakers. The GMR-based magnetic field sensor of the invention allows reducing costs by replacing the reed switch with a GMR. The GMR-based magnetic field sensor of the invention allows more reliable MR detection, reduction of part costs as well as the area required on the circuit board.

Using different connections (and thus allowing different measurement points) to a single GMR bridge can provide both features with only one component, thus keeping the costs down and limiting current consumption.

According to the invention, a resistance change is measured of a combination of shielded and unshielded GMR elements, including only shielded or only unshielded. Note: the term "shielded" refers to GMR elements having shields that reduce the magnetic field impinging on them, and "unshielded" refers to those having flux concentrators that tend to amplify magnetic fields impinging on them. The invention allows obtaining a good balance between sensitivity of a magnetic field in the least sensitive GMR element to the magnetic field orientation of no higher a field than some field strength, say 30mT, and not detecting below a value (say 1mT to 10mT) in the most sensitive orientation.

At least one GMR sensing circuit preferably is a bridge circuit comprising four GMR elements in a full bridge (Wheatstone bridge) configuration. The full bridge is formed with four GMR elements. Two of the legs, opposite each other, are shielded. The other two legs are not shielded. The shields result in a somewhat different response of the GMR to a magnetic field over a specific range. This difference results in a differential output that is often used to detect or measure the magnetic field.

The magnetic field sensor arrangement allows replacing a reed switch and adding an MRI detection function with one component instead of two components. Thus the invention provides added functionality (MRI detection) with no component cost increase.

The GMR elements preferably are resistors implemented with giant magneto-resistive material (GMR material).

The magnetic field sensor arrangement preferably comprises at least two GMR sensing circuits comprising GMR elements with different spatial orientations. A GMR has a strong sensitivity to magnetic fields aligned with its axis of sensitivity and a weak sensitivity to fields that are orthogonal to the GMRs axis of sensitivity. MRI detection in implants requires the ability to sense a field at any angle while keeping the number of required components and cost to a minimum.

Another advantage of the present invention is that problems due to saturation effects of GMR bridges with shielded and non-shielded GMR sensors are avoided. For example, such problems can manifest in a way that the differential output of a GMR bridge indicates the presence of a high magnetic field when exposed to a low magnetic field or the presence of no magnetic field when exposed to a high magnetic field in an environment of changing magnetic field strengths.

In a configuration with GMR elements arranged at different angles it is preferred that at least one GMR element has an orientation that differs from the orientation of at least one other GMR-element by a non-perpendicular angle of more the 0°.

Preferably, the GMR elements are arranged in common plane.

The magnetic field sensor arrangement preferably comprises shorting means for shorting at least one of the GMR elements. This allows to easily create a further measurement point for voltage measurement.

The shorting means preferably comprise at least one switch to short a GMR element.

The evaluation circuit preferably is configured to evaluate the relationships within the set of measured impedances by determining the differences of the measured impedances.

The object of the invention is further achieved by an implantable medical device comprising a magnetic field sensor arrangement according to invention.

Preferably, the implantable medical device is programmable and comprises a processor that is operationally connected to the magnetic field sensor arrangement.

It is further preferred that the implantable medical device is an implantable pulse generator for generating stimulation pulses.

The object of the invention is further achieved by a method for determining the presence of a magnetic field, said method comprising:
- provision of a magnetic field sensor arrangement comprising a GMR sensing circuit according to at least one of claims 1 to 9;
- measurement of the voltages between different points of the GMR sensing circuit;
- evaluation of the measured impedances and the relations of the measured impedances; and
- derivation of a signal that indicates the level of an applied magnetic field, as determined using the measured impedances and relations of the measured impedances.

The above and other aspects, features and advantages of the present invention will be more apparent from the following more particular description thereof, presented in conjunction with the following drawings wherein:
- FIG. 1: shows a dual chamber pacemaker connected to leads placed in a heart.
- FIG. 2: is a block diagram of a heart stimulator comprising a magnetic field sensor arrangement according to the invention.
- FIG. 3: shows a prior art GMR sensing circuit.
- FIGs. 4 to 8: illustrate measurement configurations that allow further voltage measurements on a GMR sensing circuit similar to the one depicted in Figure 3.
- FIGs. 9 to 15: illustrate magnetic field sensor arrangements comprising two GMR sensing circuits.
- FIGs. 16 to 22: illustrate embodiments wherein the magnetic field sensor arrangement comprises three GMR sensing circuits.
- FIG. 23: illustrates an embodiment of a magnetic field sensor arrangement comprising four GMR sensing circuits

The following description is of the best mode presently contemplated for carrying out the invention. This description is not to be taken in a limiting sense, but is made merely for the purpose of describing the general principles of the invention using an example where the implantable device is a cardiac pacemaker. The scope of the invention should be determined with reference to the claims.

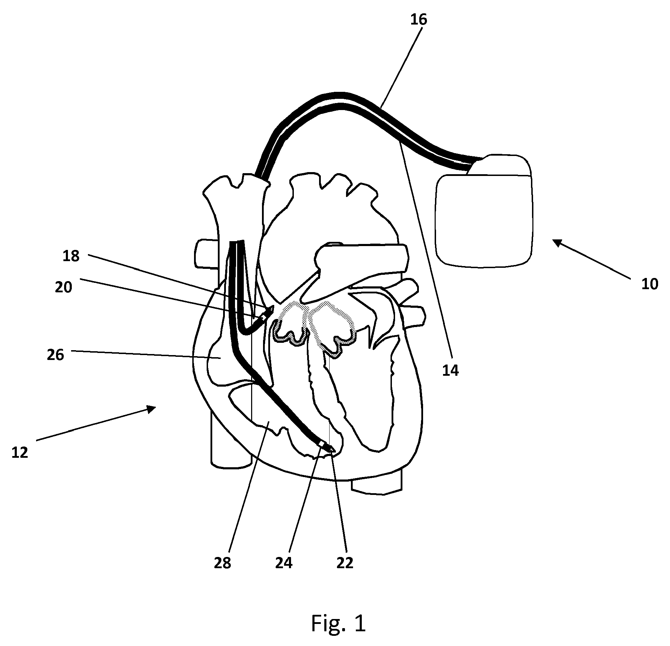

In FIG. 1 a dual chamber pacemaker 10 as heart stimulator connected to pacing/sensing leads placed in a heart 12 is illustrated. The pacemaker 10 is electrically coupled to heart 12 by way of leads 14 and 16. Lead 14 has a pair of right atrial electrodes 18 and 20 that are in contact with the right atrium 26 of the heart 12. Lead 16 has a pair of electrodes 22 and 24 that are in contact with the right ventricle 28 of heart 12. Electrodes 18 and 22 are tip-electrodes at the distal ends of leads 14 and 16, respectively. Electrode 18 is a right atrial tip electrode RA-Tip and electrode 22 is a right ventricular tip electrode 22. Electrodes 20 and 24 are ring electrodes in close proximity but electrically isolated from the respective tip electrodes 18 and 22. Electrode 20 forms a right atrial ring electrode RARing and electrode 24 forms a right ventricular ring electrode RV-Ring.

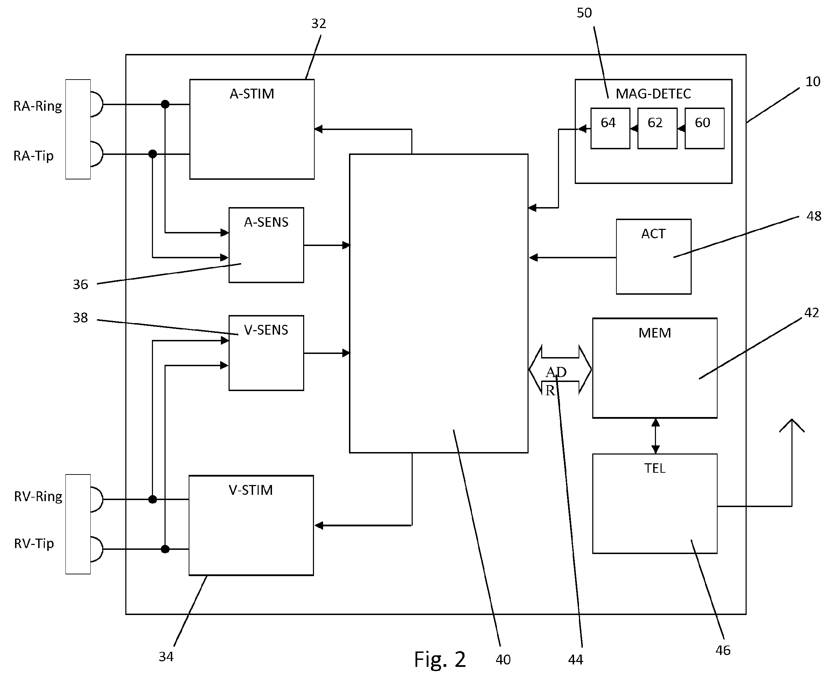

Referring to FIG. 2 a simplified block diagram of a dual chamber pacemaker 10 is illustrated. During operation of the pacemaker, leads 14 and 16 are connected to the respective output/input terminals of pacemaker 10 as indicated in Fig. 1 and carry stimulating pulses to the tip electrodes 18 and 22 from an atrial stimulation pulse generator A-STIM 32 and a ventricular pulse generator V-STIM 34, respectively. Further, electrical signals from the atrium are carried from the electrode pair 18 and 20, through the lead 14, to the input terminal of an atrial channel sensing stage A-SENS 36; and electrical signals from the ventricles are carried from the electrode pair 22 and 24, through the lead 16, to the input terminal of a ventricular sensing stage V-SENS 38.

Controlling the dual chamber pacemaker 10 is a control unit CTRL 40 that is connected to sensing stages A-SENS 36 and V-SENS 38 and to stimulation pulse generators A-STIM 32 and V-STIM 34. Control unit CTRL 40 receives the output signals from the atrial sensing stage A-SENS 36 and from the ventricular sensing stage V-SENS 38. The output signals of sensing stages A-SENS 36 and V-SENS 38 are generated each time that a P-wave representing an intrinsic atrial event or an R-wave representing an intrinsic ventricular event, respectively, is sensed within the heart 12. An As-signal is generated, when the atrial sensing stage A-SENS 36 detects a P-wave and a Vs-signal is generated, when the ventricular sensing stage V-SENS 38 detects an R-wave.

Control unit CTRL 40 also generates trigger signals that are sent to the atrial stimulation pulse generator A-STIM 32 and the ventricular stimulation pulse generator V-STIM 34, respectively. These trigger signals are generated each time that a stimulation pulse is to be generated by the respective pulse generator A-STIM 32 or V-STIM 34. The atrial trigger signal is referred to simply as the "A-pulse", and the ventricular trigger signal is referred to as the "V-pulse". During the time that either an atrial stimulation pulse or ventricular stimulation pulse is being delivered to the heart, the corresponding sensing stage, A-SENS 36 and/or V-SENS 38, is typically disabled by way of a blanking signal presented to these amplifiers from the control unit CTRL 40, respectively. This blanking action prevents the sensing stages A-SENS 36 and V-SENS 38 from becoming saturated from the relatively large stimulation pulses that are present at their input terminals during this time. This blanking action also helps prevent residual electrical signals present in the muscle tissue as a result of the pacer stimulation from being interpreted as P-waves or R-waves.

Furthermore, atrial sense events As recorded shortly after delivery of ventricular stimulation pulses during a preset time interval called the post ventricular atrial refractory period (PVARP) are generally recorded as atrial refractory sense events Ars but ignored.

Control unit CTRL 40 comprises circuitry for timing ventricular and/or atrial stimulation pulses according to a stimulation rate that can be adapted to a patient's hemodynamic need as pointed out below.

Still referring to FIG. 2, the pacemaker 10 includes a memory circuit MEM 42 that is coupled to the control unit CTRL 40 over a suitable data/address bus ADR 44. This memory circuit MEM 42 allows certain control parameters, used by the control unit CTRL 40 in controlling the operation of the pacemaker 10, to be programmably stored and modified, as required, in order to customize the pacemaker's operation to suit the needs of a particular patient. Such data includes the basic timing intervals used during operation of the pacemaker 10.

Further, data sensed during the operation of the pacemaker may be stored in the memory MEM 42 for later retrieval and analysis. This includes atrioventricular interval data that are acquired by the control unit CTRL 40. Control unit CTRL 40 is adapted to determine the atrioventricular interval data as required for automatic atrioventricular interval analysis by determining the time interval between an atrial event, either sensed (As) or stimulated (Ap), and an immediately following ventricular sensed event Vs as indicated by the ventricular sensing stage V-SENS 38.

A telemetry circuit TEL 46 is further included in the pacemaker 10. This telemetry circuit TEL 46 is connected to the control unit CTRL 40 by way of a suitable command/data bus. Telemetry circuit TEL 46 allows for wireless data exchange between the pacemaker 10 and some remote programming or analyzing device which can be part of a centralized service center serving multiple pacemakers.

The pacemaker 10 in FIG. 1 is referred to as a dual chamber pacemaker because it interfaces with both the right atrium 26 and the right ventricle 28 of the heart 12. Those portions of the pacemaker 10 that interface with the right atrium, e.g., the lead 14, the P-wave sensing stage A-SENSE 36, the atrial stimulation pulse generator A-STIM 32 and corresponding portions of the control unit CTRL 40, are commonly referred to as the atrial channel. Similarly, those portions of the pacemaker 10 that interface with the right ventricle 28, e.g., the lead 16, the R-wave sensing stage V-SENSE 38, the ventricular stimulation pulse generator V-STIM 34, and corresponding portions of the control unit CTRL 40, are commonly referred to as the ventricular channel.

In order to allow rate adaptive pacing in a DDDR or a DDIR mode, the pacemaker 10 further includes a physiological sensor ACT 48 that is connected to the control unit CTRL 40 of the pacemaker 10. While this sensor ACT 48 is illustrated in FIG. 2 as being included within the pacemaker 10, it is to be understood that the sensor may also be external to the pacemaker 10, yet still be implanted within or carried by the patient.

Control unit CTRL 40 is adapted to put the pacemaker 10 into a VOO or a DOO mode of operation wherein either only the ventricle or the ventricle and the atrium are stimulated with fixed stimulation rate and no sensing nor any inhibition is provided. For such a mode of operation, the ventricular stimulation pulse generator V-STIM 32, or both the ventricular stimulation pulse generator V-STIM 32 and the atrial stimulation pulse generator 34 can be connected to a fixed rate oscillator that is insensitive to MRI magnetic fields.

A further feature of pacemaker 10 is a magnetic field detector MAG-DETEC 50 that is connected to control unit CTRL 40. The magnetic field detector MAG-DETEC 50 is adapted to generate a detection signal characteristic of the magnetic field used for magnetic resonance imaging (MRI). The control unit CTRL 40 is adapted to process the detection signal generated by the magnetic field detector MAG-DETEC 50 and to thus positively recognize a presence of a magnetic field as used for magnetic resonance imaging (MRI). The control unit CTRL 40 responds to detection of a presence of a magnetic field as used for magnetic resonance imaging by causing the implantable medical device to enter an MRI-safe mode of operation.

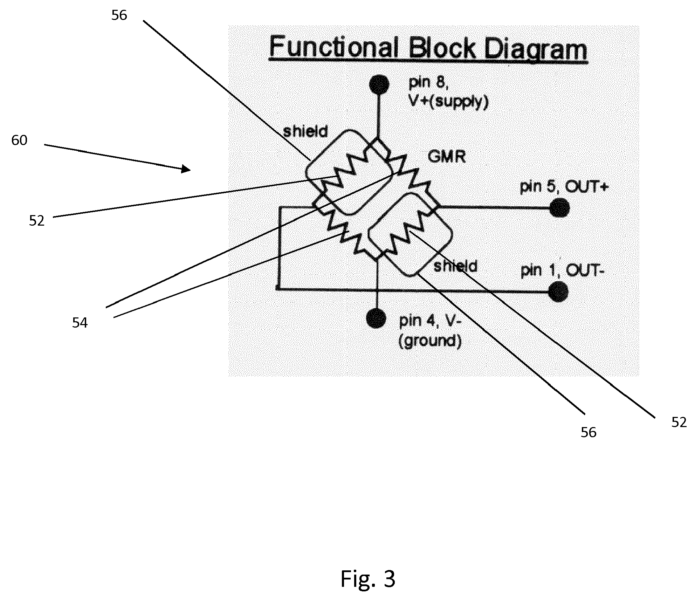

The magnetic field MAG-DETEC 50 comprises a sensor arrangement of one or more giant magneto-resistive (GMR) sensing circuits as depicted in Figures 3 to 23.

When a magnetic field is applied, the GMR effect results in a decrease in the electrical resistance of a multilayer structure comprised of alternating layers of ferromagnetic and paramagnetic thin films. A GMR sensing circuit 60 typically comprises four equal value resistors 52 and 54, fabricated using thin-film technology on a silicon substrate, in a Wheatstone bridge configuration. However, it is also possible to fabricate a GMR sensing circuit with one or more resistors.

A prior art implementation of a GMR sensing circuit 60 is shown in Figure 3. Four resistors 52 and 54 are arranged in a Wheatstone bridge configuration. The four resistors are fabricated using the same materials and with the same resistance value. Two resistors 52 have magnetic shields 56 placed over them; the other two resistors 54 are unshielded and may have flux concentrators to concentrate the magnetic field into them. The resistors are typically within the range of 2 kΩ to 50 kΩ. Due to these low resistance values, special techniques are required to reduce the average power required by the GMR control and processing circuit for use in an implantable medical device. For example, reduction of the average power may be done by using a low operating voltage or "strobing" the GMR sensor with a low duty cycle.

The behavior of the GMR sensing circuit 60 of Figure 3 depends on the strength of the magnetic field to which the GMR sensor is exposed. With no magnetic field applied and with a supply voltage applied between V- and V+, the output of the Wheatstone bridge (i.e., the voltage between OUT+ and OUT-) will be close to zero, although there may be a small offset due to the earth's magnetic field and/or resistor value mismatch. When a magnetic field is applied to the GMR sensing circuit 60 in the axis of sensitivity, the unshielded resistors will decrease in resistance as the magnetic field strength increases. The shielded resistors will also change in resistance, but by only a small amount compared to the change in the unshielded resistors over the the operating range of the GMR sensing circuit 60 in the bridge configuration.. Referring to Figure 3, this results in an increase in the voltage difference between OUT- and OUT+ when a voltage is applied between V- and V+. The change in resistance is independent of whether the field is N-S or S-N. The change in GMR resistance from that due to the background earth's magnetic field (approx. 0.05 mT) to magnetic saturation of the device results in a typical resistance change of 12% to 25%.

The GMR sensing circuit in Figure 3 is sensitive to the axis of the magnetic field, as is also the case with a reed switch. The "window" where the orientation of the applied magnet axis results in the reed switch opening is similar to that of a GMR sensing circuit in a practical application.

GMR sensing circuits are comprised of GMR elements that are electronic resistors that change their resistance as a function of a magnetic field impinging on them. In general, the stronger the magnetic field, the lower the devices resistance.

GMR elements are sensitive to the orientation of the magnetic field with respect to the GMR element. Rotating a GMR element 90 degrees in a fixed magnetic shield can result in a very significant change in the GMR element's resistance.

The change in resistance in a GMR element can be modified by shielding it with either a material that reduces the magnetic field impinging on the GMR element, or a material that increases the magnetic field impinging on the GMR element.

GMR sensing circuits may be designed to detect or measure low level magnetic fields. These are typically an arrangement of four GMR elements into a bridge configuration (see Figure 3). Two opposite legs of the bridge are shielded and the other two are not. When the bridge is stimulated, the output differential voltage is proportional to the magnetic field impinging on it with in a limited range and within a limited orientation of the magnetic field and the GMR elements 52 and 54 themselves. Both of these materials and approaches are referred to as "shielding" in this document.

When looking at the overall resistance of these devices for use in higher magnetic field detection circuits, such as MRI static field detection it was discovered that there is a significant difference in the resistance change of the shielded vs. the unshielded GMR elements with respect to the impinging field strength and with respect to the orientation of the magnetic shield with respect to the GMR element.

In one orientation a shielded GMR element gives better guaranteed detection at, for example, 30mT. But when rotated 90 degrees, it may provide very poor guaranteed nondetection below a lower limit, say 10mT. Shielding of the same GMR element can modify this behavior. Thus by using a combination of shielded and unshielded GMR elements a better balance between the two needs can be achieved.

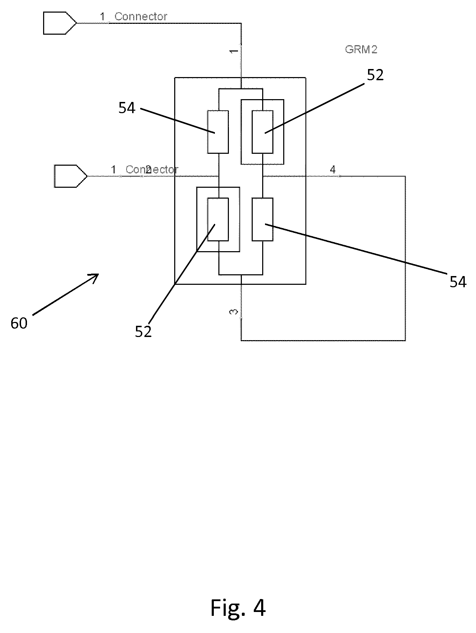

Because GMR elements are sensitive to the orientation of an impinging magnetic field, prior art GMR sensor arrangements may not be able to reliably detect both high and low magnetic fields with different orientations. Therefore, the magnetic field detector 50 comprises a magnetic field sensor arrangement with a GMR sensing circuit that allows voltage measurement not only across the bridge (as in Figure 3), but at further measurement points of the GMR sensing circuit. One way of selectively creating further measurement points is to selectively short one or more GMR elements. Figures 4 to 8 illustrate measurement configurations that allow further voltage measurements on a GMR sensing circuit similar to the one depicted in Figure 3. Thus, a plurality of measurement configurations are made possible that allow various voltage measurements that can be evaluated absolutely and in relation to each other.

As can be seen in Figure 4, one measurement configuration is to measure the impedance between the terminals of one unshielded GMR element (resistor 54) while the other unshielded GMR element (the other resistor 54) is shorted.

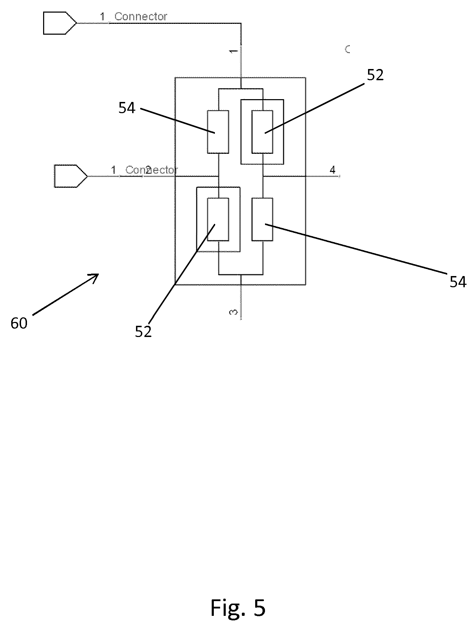

Figure 5 depicts a further measurement configuration that differs from the configuration in Figure 4 in that the second unshielded GMR element 54 is not shorted.

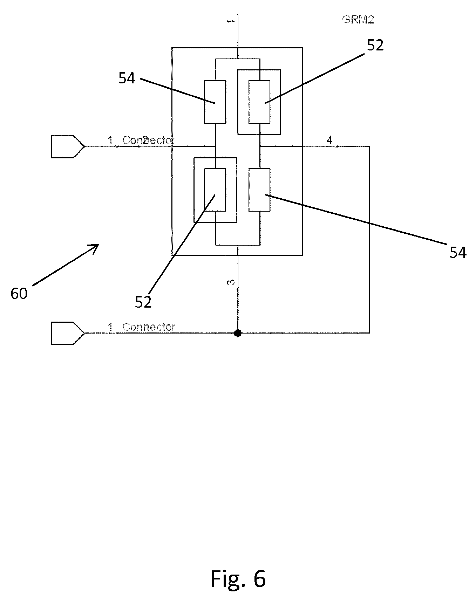

Figure 6 discloses yet another measurement arrangement wherein the second unshielded GMR element (resistor 54) is shorted and voltage measurement is carried out over a shielded GMR element 52 (shielded resistor 52).

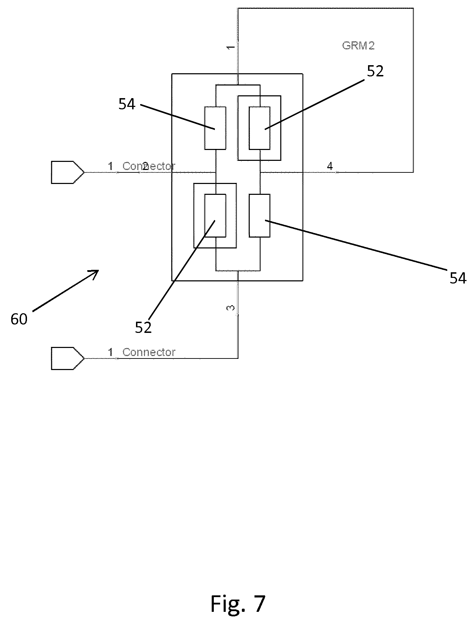

In Figure 7, yet another measurement configuration is depicted wherein a voltage measurement is made across one shielded GMR element 52 (resistor 52) while the other shielded GMR element 52 (the other shielded resistor 52) is shorted.

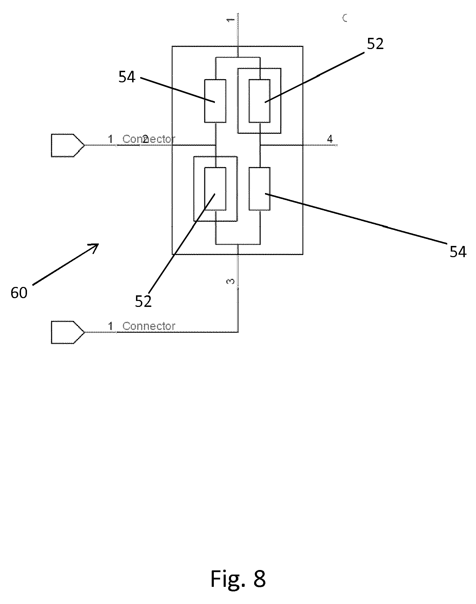

Figure 8 illustrates yet another measurement configuration wherein measurement is again carried out over one shielded GMR element 52 (shielded resistor 52) while the other shielded GMR element 52 is not shorted (as opposed to the arrangement in Figure 6).

Thus, Figures 3 to 8 illustrate several of the voltage measurement configurations which provide for different measured voltages that can be evaluated in an absolute manner and in relation to each other.

Preferably, the evaluation circuit 64 is configured to provide a relative evaluation of voltages by generating differences between measured voltages.

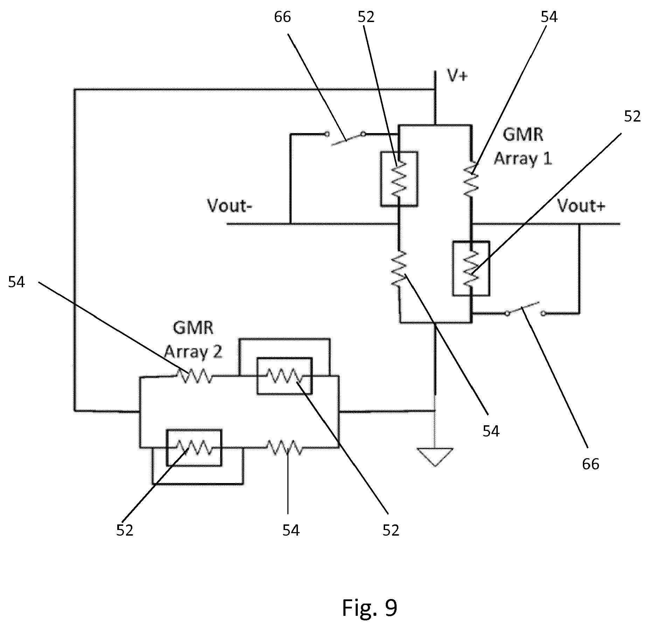

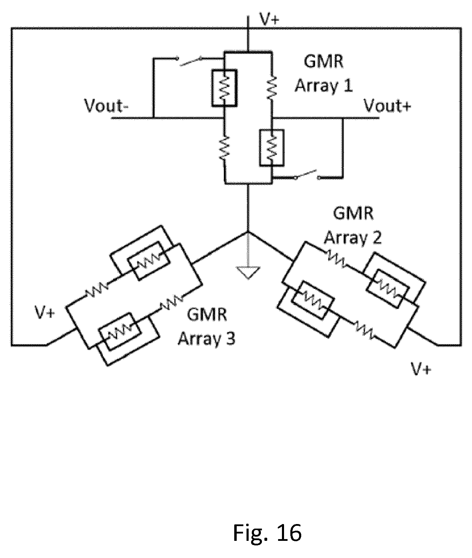

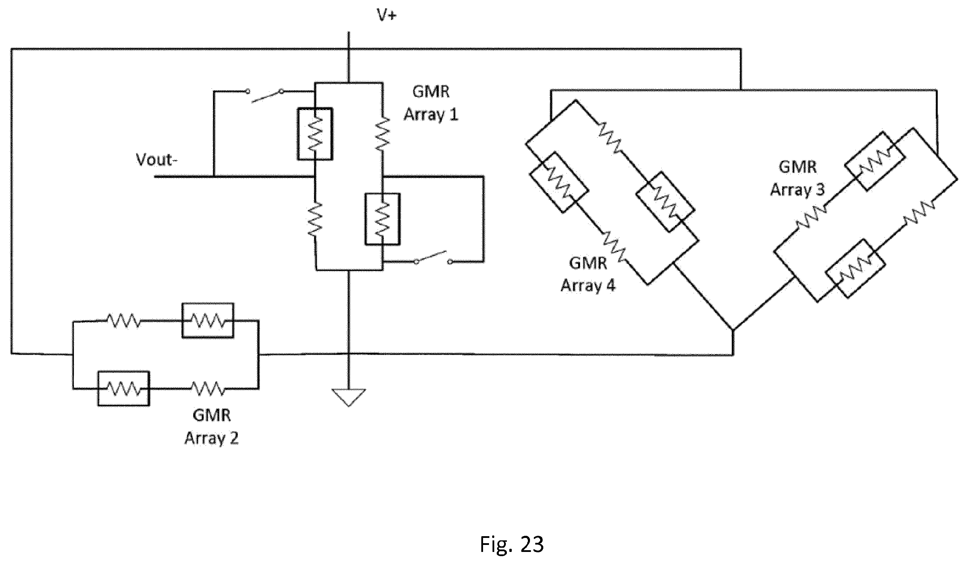

Figures 9 to 23 illustrate further examples of magnetic field sensor arrangements that are comprised of more than one GMR sensing circuit. In particular, Fig. 9 to 15 illustrate magnetic field sensor arrangements comprising two GMR sensing circuits and Fig. 16 to 22 illustrate embodiments wherein the magnetic field sensor arrangement comprises three GMR sensing circuits. Finally, Fig. 23 illustrates an embodiment of a magnetic field sensor arrangement comprising four GMR sensing circuits.

Fig. 9 to 23 are schematic representations wherein the GMR sensing circuits are shown at various angles to each other. These orientations represent the mechanical orientations of the GMR sensing circuits relative to each other for each specific magnetic field sensor arrangement. If two or more GMR sensing circuits are combined to form a magnetic field sensor arrangement, the sensitivity of the overall magnetic field sensor arrangement to the orientation of an impinging magnetic field can be decreased. Thus, the magnetic field sensor arrangement will provide more uniform results, depending only on the strength of the magnetic field rather than on the orientation of an impinging magnetic field.

These example configurations combine several of the properties of the invention disclosure in a useful manner.

In Fig. 9 to 23 the individual GMR sensing circuits are designated as "GMR array". The embodiment depicted in Fig. 9 is a magnetic field sensor arrangement comprising two GMR sensing circuits.

The two GMR sensing circuits are orientated at 90 degrees relative to each other. Each GMR sensing circuit (GMR array 1 and GMR array 2) is comprised of two unshielded GMR elements and two shielded GMR elements. In the second GMR sensing circuit (GMR array 2) the two shielded GMR elements are shorted and thus ineffective. The same effect could also be achieved with a GMR sensing circuit comprising only one unshielded GMR element.

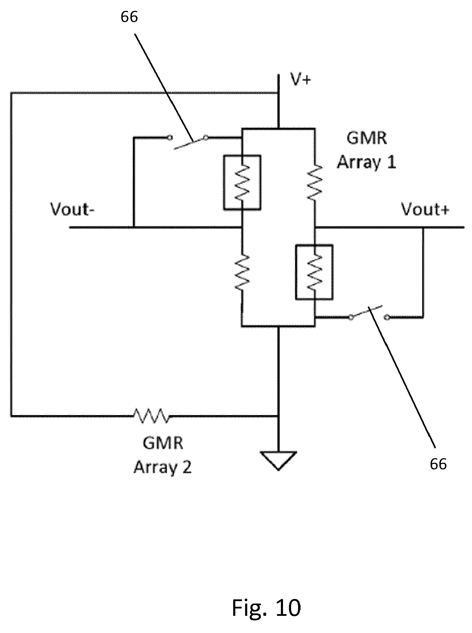

With respect to the first GMR sensing circuit (GMR array 1), the embodiments of Figure 9 and 10 are identical. As can be seen, switches 66 are provided that allow selectively shorting the shielded GMR elements in the first GMR sensing circuit. By way of switches 66, the first GMR sensing circuit (GMR array 1) may be configured to be used as a bridge (similar to the one shown in Figure 3) or for bulk resistance measurement, depending on the application.

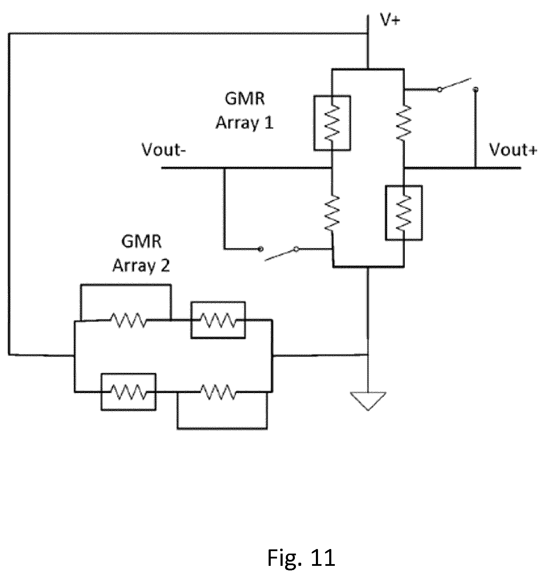

The embodiment of Figure 11 is similar to the embodiment of Figure 9. The difference with respect to the embodiment of Figure 9 is that in the first GMR sensing circuit (GMR array 1) switches 66 are provided that allow shorting of the unshielded GMR elements of the first GMR sensing circuit. Similarly, in the second GMR sensing circuit (GMR array 2) of the embodiment of Figure 11, both unshielded GMR sensing elements are shorted and thus ineffective.

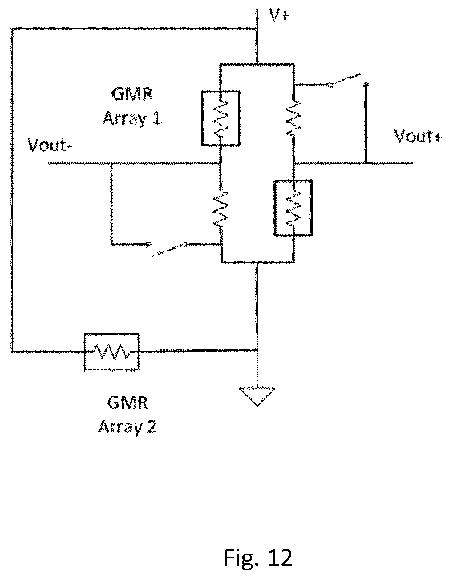

Figure 12 depicts an embodiment that is equivalent to the embodiment of Figure 11. In the embodiment of Figure 12, both effective shielded GMR elements of the second GMR sensing circuit are replaced by a single shielded GMR element.

Again, switches 66 in the embodiments of Figures 11 and 12 allow using the first GMR sensing circuit (GMR array 1) as a bridge or for bulk resistance measurement, respectively, depending on whether or not switches 66 are open or closed, respectively.

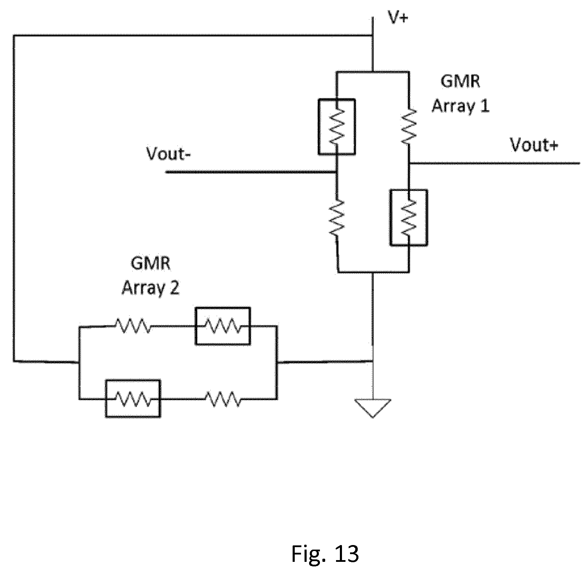

Figure 13 illustrates an embodiment with two GMR sensing circuits (two GMR arrays) arranged at 90 degrees with respect to each other and with no shorted GMR elements. The first GMR sensing circuit of the embodiment of Figure 13 is used as a traditional full bridge as in Figure 3 whereas the second GMR sensing circuit (GMR array 2) is configured for bulk resistance measurement.

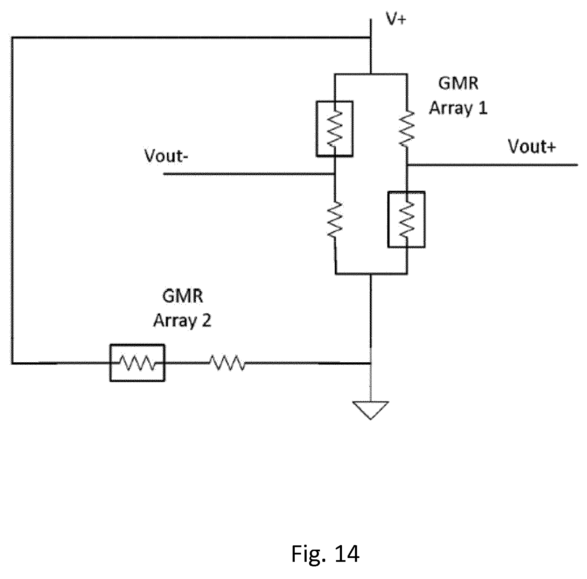

Again, the same effect achieved by the embodiment of Figure 13 can be achieved with a more simple second GMR sensing circuit. Figure 14 discloses a simplified second GMR sensing circuit equivalent to the second GMR sensing circuit in the embodiment of Figure 13. In the embodiment of Figure 14, the second GMR sensing circuit comprises one shielded GMR element and one unshielded GMR element connected in series.

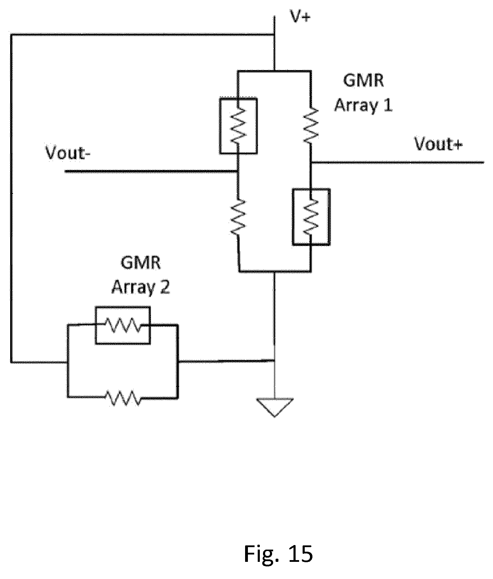

Alternatively, the effect of the second GMR sensing circuit of the embodiment of Figure 13 can be achieved with a second GMR sensing circuit comprising one shielded GMR element and one unshielded GMR element connected in parallel to each other; cf. Figure 15.

While the use of two GMR sensing circuits in combination with each other will reduce the sensitivity of the magnetic field sensor arrangement with respect to the special orientation of an impinging magnetic field, that advantage comes at the costs of higher complexity and power drain.
In particular, the embodiments of Figures 10, 12, 14 and 15 provide for an acceptable behavior at acceptable expenses, as opposed to the embodiments of Figures 9 and 11..

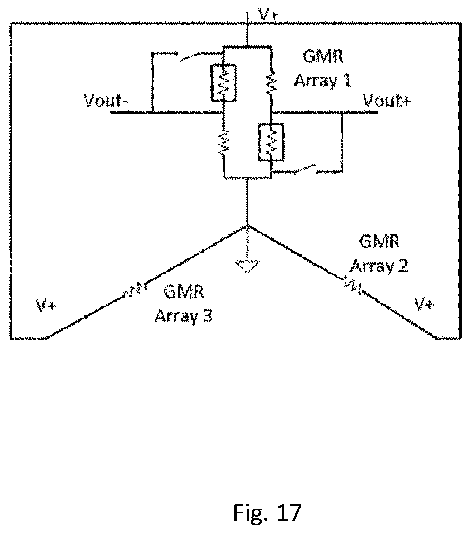

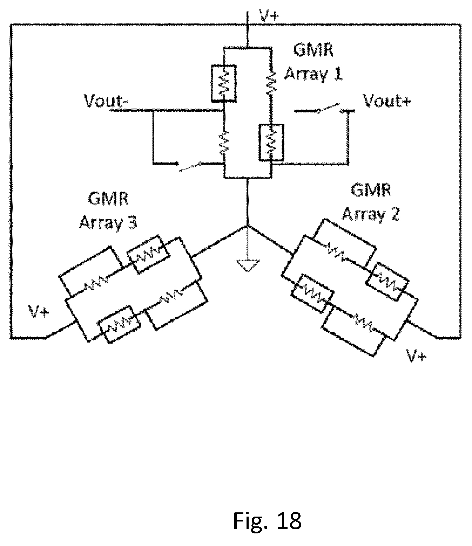

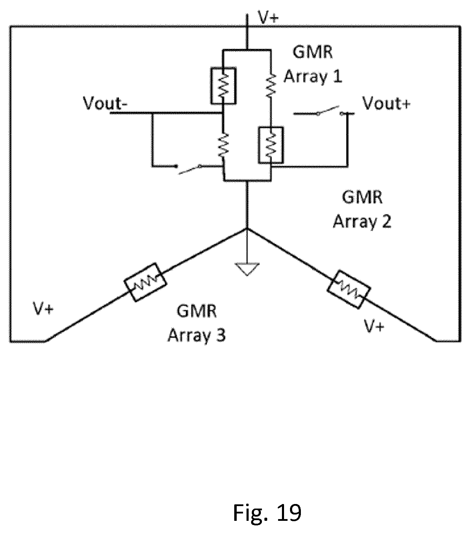

Figure 16 to 22 each disclose different embodiments of a magnetic field sensor arrangement comprising three GMR sensing circuits (three GMR arrays) with different spatial orientations. In particular, the three GMR sensing circuits of each of the embodiments of Figures 16 to 22 are spatially oriented at 0°, 60° and 120°. As in the embodiments of Figures 9 to 12, the first GMR sensing circuit (GMR array 1) comprises switches 66 for selectively shorting one or more of the GMR elements, thus allowing a voltage measurement that corresponds either to the traditional Wheatstone bridge arrangement or to a bulk voltage measurement. With respect to the second and the third GMR sensing circuits of the embodiments of Figures 16 to 22, it is noted that these may either comprise shorted GMR elements (see Figures 16 and 18), or they may be replaced by just a single GMR element (see Figures 17 and 19), in a manner similar to the embodiments of Figures 10 and 12.

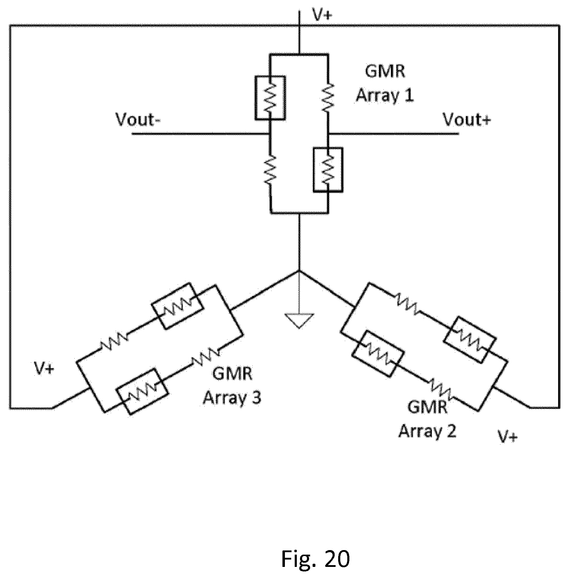

Likewise, the magnetic field sensor arrangement may comprise no switches, as in the embodiments of Figures 20 to 22.

In the embodiments of Figures 20 to 22, the first GMR sensing circuit (GMR array 1) is connected in a traditional Wheatstone bridge arrangement similar to the embodiment of Figure 3 or of Figures 13 to 15.

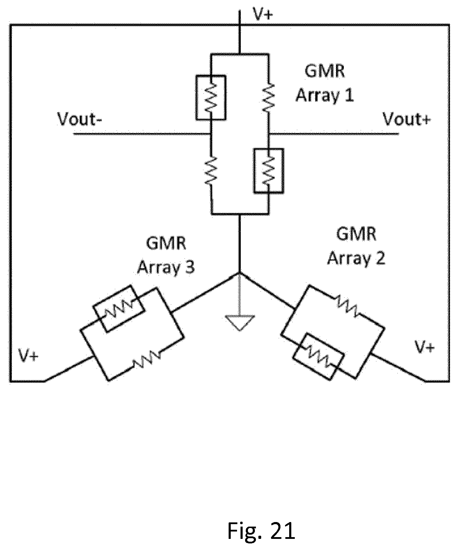

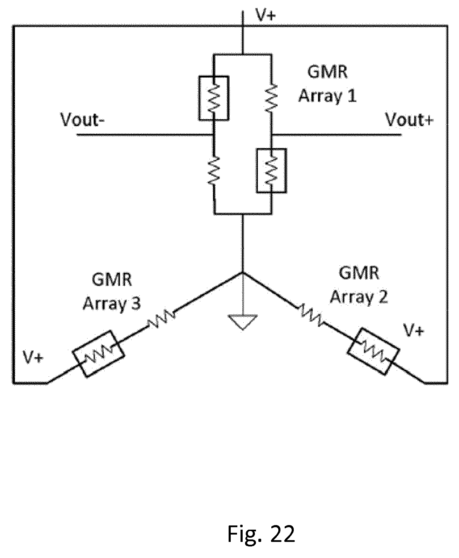

Further, none of the elements of GMR Array 1 is shorted in the embodiments of Figures 20 to 22. As can be seen in Figures 21 and 22, the second and the third GMR sensing circuits can be simplified and replaced by GMR sensing circuit comprising only one shielded and one unshielded GMR element similar to the embodiments of Figures 14 and 15.

The first GMR sensing circuit can be configured for use as a magnet detect or for an MRI field detect. The three GMR sensing circuits are arranged with equal angular spacing so that, together, they provide a much more uniform MRI response with respect to angle than a single GMR or 2 GMRs at 90 degrees.

Shorting out the shielded legs of a GMR sensing circuit (GMR array) yields a device that has high sensitivity at low fields and lower sensitivity at high fields. Shorting out the unshielded would give a device with low sensitivity at low fields and moderate sensitivity at higher fields.

A magnetic field sensor arrangement comprising three GMR sensing circuits is considered an optimum between very good response and cost. Note: Using three identical GMR sensing circuits helps to ensure behavior matching, which is important when using these devices in parallel like this.

This approach can be used with any number of arrays greater than 1, but the use of 2, 3 or 4 arrays may provide the most cost efficient, and thus the most useful configurations.

Even less sensitivity to magnetic field orientation can be achieved with a magnetic field sensor arrangement comprising four GMR sensing circuits as in the embodiment of Figure 23. In the embodiment of Figure 23, four GMR sensing circuits are provided that are spatially oriented at 0 degrees, 45 degrees, 90 degrees and 145 degrees. One of the GMR sensing circuits (GMR array 1) comprises switches for selective shorting its shielded GMR elements.

For each of the four GMR sensing circuits of the embodiment of Figures 23, the same operations are possible as illustrated with respect to the previous embodiment in Figures 9 to 23.

This approach presented herein requires fewer components, thus lower cost, than other solutions. It also may require less power and thus save battery life over multiple component solutions.

Although exemplary embodiments of the present invention have been shown and described, it should be apparent to those of ordinary skill in the art that a number of changes and modifications to the invention may be made without departing from the spirit and scope of the present invention. This invention can readily be adapted to a number of different kinds of applications in the field of magnetic field detection by following the present teachings. All such changes, modifications and alterations should therefore be recognized as falling within the scope of the present invention.
- 10: pacemaker
- 12: heart
- 14, 16: lead
- 18, 20: atrial tip electrodes
- 22, 24: ring electrodes
- 26: right atria
- 28: right ventricle
- 32: atrial stimulation pulse generator A-STIM
- 34: ventricular stimulation pulse generator V-STIM
- 36: sensing stage A-SENS
- 38: ventricular sensing stage V-SENS
- 40: control unit CTRL
- 42: memory circuit MEM
- 44: data/address bus ADR
- 46: telemetry circuit TEL
- 48: physiological sensor ACT
- 50: magnetic field detector MAG-DETEC
- 52, 54: GMR elements
- 56: magnetic shields
- 60: GMR sensing circuit
- 62: measurement circuit
- 64: evaluation circuit
- 66: switches

## Claims

1. Magnetic field sensor arrangement (50) comprising at least one Giant magnetoresistance (GMR) sensing circuit (60) comprising GMR elements (52,54), wherein at least one of the GMR elements (52) is at least partially housed in a shielding or flux concentrating first material that reduces or amplifies, respectively, the magnetic field impinging on it, while at least one other GMR element (54) is not housed in such a material or is housed in a material having an opposite effect than the first material, said magnetic field sensor (50) further comprising a measurement circuit (62) that is configured for the voltage measurement at different points of the GMR sensing circuit (60) for deriving an electric impedance between the different measurement points to derive a signal that indicates a level of an outer magnetic field.

2. Magnetic field sensor arrangement (50) according to claim 1, further comprising an evaluation circuit (64) that is configured to evaluate the measured impedances and the relations of the measured impedances to derive a signal that indicates a level of an outer magnetic field.

3. Magnetic field sensor arrangement (50) according to claim 1 or 2 wherein at least one GMR sensing circuit (60) is a bridge circuit comprising four GMR elements (52, 54) in a full bridge (Wheatstone bridge) configuration.

4. Magnetic field sensor arrangement (50) according to at least one of the preceding claims, wherein the GMR elements (52, 54) are resistors implemented with giant magneto-resistive material.

5. Magnetic field sensor arrangement (50) according to at least one of the preceding claims comprising at least two GMR sensing circuits (60), each comprising GMR elements (52, 54), the GMR sensing circuits having different spatial orientations.

6. Magnetic field sensor arrangement (50) according to claim 5, wherein at least one GMR element has a spatial orientation that differs from the orientation of at least one other GMR element by a non-perpendicular angle of more than 0°.

7. Magnetic field sensor arrangement (50) according to claim 5 or 6 wherein the GMR elements (52, 54) are arranged in a common spatial plane.

8. Magnetic field sensor arrangement (50) according to at least one of claims 1 to 7 comprising means for electrically shorting at least one of the GMR elements (52, 54).

9. Magnetic field sensor arrangement (50) according to claim 8, wherein the shorting means comprises at least one switch (66) to short a GMR element.

10. Magnetic field sensor arrangement (50) according to at least one of claims 1 to 9 wherein the evaluation circuit (64) is configured to evaluate the relation of the measured impedances by determining the differences between the measured impedances.

11. Implantable medical device comprising a magnetic field sensor arrangement according to at least one of claims 1 to 10.

12. Implantable medical device according to claim 11, wherein said implantable medical device is programmable and comprises a processor that is operationally connected to the magnetic field sensor arrangement (50).

13. Implantable medical device according to claim 11 or 12 wherein said implantable medical device is an implantable pulse generator for generating stimulation pulses.

14. A method for determining the presence of a magnetic field, said method comprising:
- provision of a magnetic field sensor arrangement (50) comprising a GMR sensing circuit (60) according to at least one of claims 1 to 10;
- measurement of the voltages between different points of the GMR sensing circuit (60);
- evaluation of the measured impedances and the relations of the measured impedances; and
- derivation of a signal that indicates the level of an applied magnetic field, as determined using the measured impedances and relations of the measured impedances.
